# EUROPÄISCHE PATENTSCHRIFT

(11) **EP 2 216 326 B1**
(45) Veröffentlichungstag und Bekanntmachung des Hinweises auf die Patenterteilung: **25.09.2013**
(21) Anmeldenummer: 10152626.7
(22) Anmeldetag: 04.02.2010
(51) Int. Cl.: C07C 305/04, C07C 215/90, C11D 1/29

(54) **Biologisch verträgliche Cholinverbindungen und deren Verwendung als Tenside**
Biologically acceptable choline compounds and their use as tensides
Composés de choline biologiquement acceptables et leur utilisation en tant que tensioactif

(30) Priorität: 04.02.2009 DE 102009007443; 29.05.2009 DE 102009026598
(43) Veröffentlichungstag der Anmeldung: 11.08.2010
(73) Patentinhaber: Universität Regensburg, 93053 Regensburg (DE)
(72) Erfinder: Prof. Dr. Kunz, Werner, 93051 Regensburg (DE); Kellermeier, Matthias, 78464 Konstanz (DE); Klein, Regina, 67346 Speyer (DE); Maurer, Eva, 93073 Neutraubling (DE); Dr. Touraud, Didier, 93051, Regensburg (DE)
(74) Vertreter: HOFFMANN EITLE

(56) Entgegenhaltungen:
- WO-A1-99/43775
- DATABASE CA [Online] CHEMICAL ABSTRACTS SERVICE, COLUMBUS, OHIO, US; LI, DUXIN ET AL: "Study on synthesis and application of .beta.-hydroxyethyltrimethylammonium dodecyl sulfate", XP002675706, gefunden im STN Database accession no. 1997:234979

## Beschreibung

Seifen bzw. Tenside sind allgegenwärtig im täglichen Leben, zum Beispiel als Emulgatoren nicht nur in Haushaltsreinigungsmitteln und Körperpflegeprodukten, sondern auch in Nahrungsmitteln. Die gebräuchlichsten Seifen, bekannt seit über 1000 Jahren, sind die Natrium- und Kaliumsalze der Fettsäuren. Der Vorteil dieser klassischen Carboxylatseifen sind ihre niedrigen Herstellungskosten sowie ihre biologische Verträglichkeit. Als Nachteil lässt sich die begrenzte Wasserlöslichkeit anführen, die bei einem pH-Wert unterhalb von etwa 9 oder auch in Gegenwart von Salzen wie NaCl weiter herabgesetzt ist.

Die Salzempfindlichkeit lässt sich anhand der Erhöhung der Löslichkeitstemperatur (Krafft-Temperatur) in Abhängigkeit von der Salzkonzentration quantifizieren. Insbesondere verringert sich die Löslichkeit der Carboxylatseifen in Gegenwart von Salzen zweiwertiger Kationen wie Calcium oder Magnesium (Härtebildner). Um ein Ausfallen der Seifen zu vermeiden, müssen in der Regel Wasserenthärter zugesetzt werden, die wiederum oft umweltschädlich sind.

Natriumalkylsulfate und -sulfonate stellen eine effiziente Alternative zu den Fettsäureseifen dar. Sie sind weniger salzempfindlich, sind gut biologisch abbaubar und können aus erneuerbaren Materialien gewonnen werden. Der bekannteste und am besten untersuchte Vertreter dieser Klasse von Tensiden ist Natriumdodecylsulfat (SDS), das häufig in der Formulierung von z.B. Körperpflegeprodukten wie Shampoos oder Duschgels eingesetzt wird. Reines SDS löst sich in Wasser typischerweise bei Temperaturen oberhalb von 12°C bis 25°C. Obwohl es weniger salzempfindlich ist als klassische Fettsäureseifen, übersteigt die Löslichkeitstemperatur von SDS bei Zugabe von Salz dennoch schnell 25°C. Darüberhinaus ist der Gebrauch der oberflächenaktiveren längerkettigen Homologen von SDS, wie zum Beispiel Natriumtetradecylsulfat, limitiert aufgrund der geringen Wasserlöslichkeit.

Aus diesem Grund werden Alkylsulfate in der Praxis oft durch Alkylethersulfate ersetzt. Die Einführung von Oxyethylengruppen in die Alkylketten verbessert die Wasserlöslichkeit und verringert die Salzempfindlichkeit merklich. Weiterhin ist bei Alkylethersulfat-Tensiden die Hautreizung im Vergleich zu einfachen Alkylsulfaten geringer. Dementsprechend häufig sind Alkylethersulfate in Flüssigformulierungen zu finden, besonders in kosmetischen Produkten wie Lotionen, Cremes, Shampoos und Duschgelen, aber auch in flüssigen Haushaltsreinigern und Spülmitteln. Handelsübliche Vertreter der Alkylethersulfat-Tenside sind die Natriumsalze von Sulfaten ethoxylierter Kokosöl-Alkohole (12-14 C-Atome) mit einem typischen Ethoxylierungsgrad von 2-3. Am bedeutendsten ist dabei Natriumlaurylethersulfat (LES), welches großtechnisch produziert wird und in diversen Formulierungen eine breite Anwendung findet.

Ein Hauptnachteil der Alkylethersulfate liegt in deutlich erhöhten Produktionskosten verglichen mit einfachen Alkylsulfaten. Außerdem werden zur Synthese von Alkylethersulfaten Ethylen- bzw. Polyethylenglykole eingesetzt, deren Herstellung wiederum auf dem toxischen und hochentzündlichen Gas Ethylenoxid basiert, und die Spuren des karzinogenen 1,4-Dioxans enthalten können.

Es ist weiterhin bekannt, dass die Löslichkeit von anionischen Tensiden wie Carboxylaten oder gegebenenfalls alkoxylierten Alkylsulfat- oder -sulfonat-Tensiden durch das Gegenion beeinflusst wird. So steigen die Krafft-Punkte der Sulfat- oder Sulfonat-Tenside mit zunehmender Größe des Gegenions in der Reihe Li⁺, Na⁺, K⁺, Rb⁺, Cs⁺, während bei den Carboxylaten der umgekehrte Trend beobachtet wird (M. J. Rosen, Surfactants and Interfacial Phenomena, 2nd Ed., John Wiley & Sons Ltd., USA, 1989, pp. 216 - 217). Somit führen kleine, stark solvatisierte Kationen zu einer Erhöhung der Löslichkeit von Sulfat- oder Sulfonat-Tensiden. Bei größeren Ionen wäre eine niedrigere Wasserlöslichkeit zu erwarten.

Überraschenderweise wurden jedoch auch bestimmte größere Kationen wie Tetramethylammonium gefunden, die die Löslichkeit von Sulfat- oder Sulfonat-Tensiden erhöhen können. So konnte zum Beispiel gezeigt werden, dass Tetramethylammoniumdodecylsulfat wasserlöslich ist bis unter 0°C, wobei zugleich die Empfindlichkeit gegenüber der Zugabe von beispielsweise Natriumchlorid weniger stark ausgeprägt ist (Yu et al., J. Phys. Chem. 1990, 94, 3675; Mukerjee et al., J. Phys. Chem. 1958, 62(11), 1400; Berr et al. J. Phys. Chem. 1986 90(24), 6492; Berrau et al. J. Phys. Chem. B 2003, 107, 13432). Der praktische Einsatz von Tetramethylammonium anstelle von Alkali-Ionen ist allerdings stark limitiert, da einfache Tetraalkylammonium-Ionen giftig sind. Ihre Toxizität beruht unter anderem darauf, dass sie als Phasentransferkatalysatoren fungieren können, oder auf der Fähigkeit, zelluläre Ionenkanäle zu blockieren.

Als Maßnahme zum Erhöhen der Wasserlöslichkeit klassischer Carboxylatseifen wird in R. Klein, D. Touraud, W. Kunz, Green Chemistry, 2008, 10, 433 vorgeschlagen, als Gegenion Cholin ((2-Hydroxyethyl)trimethylammonium) anstelle von Natrium oder Kalium einzusetzen, das ungiftig ist und die Krafft-Temperatur, also die Temperatur, ab der das Tensid in wässriger Lösung Micellen bilden kann, im Vergleich zu Natrium um 30 bis 45°C verringert. Im Gegensatz zu den Sulfaten oder Sulfonaten erhöht sich bei den Carboxylaten die Löslichkeit mit zunehmender Größe des Kations, so dass dieses Ergebnis nicht überrascht.

Cholincarboxylate haben jedoch den Nachteil, dass die Salzempfindlichkeit, und insbesondere die Empfindlichkeit gegenüber hoher Wasserhärte, höher ist als beispielsweise bei SDS. Ein weiterer Nachteil ist die hohe Empfindlichkeit in Bezug auf den pH-Wert. Es ist nicht möglich, Carboxylat-Tenside bereitzustellen, die bei einem hautneutralen pH-Wert von 5 noch löslich sind, was daran liegt, dass Fettsäuren im Gegensatz zu Alkylsulfon- oder -schwefelsäuren nur sehr schwache Säuren sind. Dementsprechend haben reine Carboxylatseifenlösungen einen pH-Wert von 10-12. Daher werden die Fettsäuren oft nur halbneutralisiert, um einen pH-Wert von 8 - 9 zu erreichen und damit die Hautverträglichkeit zu gewährleisten. Mit Absenkung des pH-Wertes steigen allerdings die Krafft Temperaturen stark an, da die reinen neutralisierten Fettsäuren gänzlich wasserunlöslich sind. Nichtsdestoweniger wirkt selbst ein pH-Wert von 8 - 9 auf die Haut noch reizend und austrocknend.

Schließlich sind Cholincarboxylate auch sehr hygroskopisch, was eine Aufbewahrung und Verarbeitung unter wasserfreier Atmosphäre erforderlich macht. Bei Lagerung an Luft ziehen sie Luftfeuchtigkeit an und gehen dabei in lyotrope, flüssigkristalline Phasen über, welche von der Konsistenz her gelartig und klebrig sind.

β-Hydroxyethyltrimethylammoniumdodecylsulfat war zur Verwendung als Wachstumsregulator zur Steigerung der Ausbeute an Weizen im landwirtschaftlichen Bereich bekannt (Database CA, Chemical Abstract Service, Columbus, Ohio, US; Li, Duxin et al.: "Study on Synthesis and application of beta-hydroxyethyltrimethylammoniumdodecyl sulfate", Stn, XP02675706, Database accession no. 1997:234979).

WO 99/43775 A1 offenbart quaternäre Fettsäuretriethanolaminestersalze zur Verwendung als Tenside.

Der vorliegenden Erfindung liegt somit die Aufgabenstellung zugrunde, ein Tensid bereitzustellen, das die Nachteile der bekannten Tenside des Standes der Technik vermeidet und das eine hohe Löslichkeit und Salzresistenz aufweist und gut hautverträglich ist.

Erfindungsgemäß wird diese Aufgabe durch Cholinverbindungen der folgenden Formel:

R-A-SO₃⁻ [(H₃C)₃N-CH₂-CH₂-OH]⁺

gelöst, worin A für ein Sauerstoffatom oder eine Gruppe der Formel -[O-B]ₙ-O- steht, wobei B eine Alkylengruppe mit 2 bis 4 Kohlenstoffatomen darstellt und n für eine Zahl von 1-20 steht; und R für eine gesättigte oder ungesättigte, lineare oder verzweigte Alkylgruppe mit 8 bis 30 Kohlenstoffatomen steht, wobei β-Hydroxyethyltrimethylammoniumdodecylsulfat ausgenommen ist.

Bevorzugterweise hat R 8 bis 24, insbesondere 8 bis 20 Kohlenstoffatome. Weiterhin sind lineare, gesättigte oder einbis dreifach ungesättigte Alkylgruppen bevorzugt. Insbesondere bevorzugt sind gesättigte oder einfach ungesättigte, lineare Alkylgruppen mit 12 bis 18 Kohlenstoffatomen, z.B. n-Dodecyl oder n-Tetradecyl.

B ist bevorzugterweise eine Ethylengruppe -CH₂-CH₂-, eine 2-Propylengruppe -CH(CH₃)-CH₂- oder eine Mischung daraus.

Als Beispiele für bevorzugte erfindungsgemäße Verbindungen lassen sich somit Cholin-Fettalkoholsulfate anführen. Besonders bevorzugt sind Cholindodecylsulfat (ChDS) oder Cholintetradecylsulfat (ChTS).

Aufgrund ihrer erhöhten Oberflächenaktivität sind Verbindungen, in denen R 14 oder mehr, z.B. 14 bis 24 Kohlenstoffatome hat, ebenfalls bevorzugt. Als Beispiele lassen sich Cholincetylsulfat, Cholinstearylsulfat, Cholinoleylsulfat, Cholineicosylsulfat oder Cholinbehenylsulfat anführen.

Cholin, früher bekannt als Vitamin B4, besitzt diverse Schlüsselfunktionen im menschlichen Körper (z.B. als Vorstufe für Phosphorlipide oder für den Neurotransmitter Acetylcholin) und ist in vielen Nahrungsmitteln enthalten. Im Vergleich zu konventionellen quaternären Ammonium-Ionen ist Cholin gut biologisch abbaubar, was vermutlich auf die Hydroxyl-Gruppe zurückzuführen ist. Wie in G. J. J. Kortstee, Arch. Microbiol., 1970, 71, 235 gezeigt, kann Cholin.beispielsweise durch Mikroorganismen abgebaut werden.

Während konventionelle quaternäre Ammoniumionen hochtoxisch sind, ist Cholin ein körpereigener Metabolit. In Ratten-Gliom-Zellen weist Cholin im Vergleich zu Tetramethylammonium (TMA) eine um den Faktor 5,5 geringere Toxizität auf. Im Vergleich zu Tetrabutylammonium ist die Toxizität über 500-fach geringer (L. C. Mokrasch, Mol. Cell. Biochem. , 1990, 92, 86).

Zudem gilt Cholin allgemein als stark feuchtigkeitsspendend bei der Behandlung von Haut oder Haar und wurde erfolgreich zur Reduzierung von Brennen bzw. Irritationen der Haut in Formulierungen eingesetzt (US 2002-010113). Dementsprechend wirken auch die erfindungsgemäßen Cholinverbindungen weniger reizend als beispielsweise SDS, welches insbesondere in Reinform oder in konzentrierter Lösung reizend sowie potentiell auch allergen auf Haut und Augen wirkt.

Die Verbindungen der vorliegenden Erfindung, einschließlich β-Hydroxyethyltrimethylammoniumdodecylsulfat, lassen sich erfindungsgemäß als Tenside einsetzen, beispielsweise in Körperpflegeprodukten wie Duschgelen, in Haarpflegeprodukten wie Shampoos oder Conditioner oder in Hautpflegeprodukten. Wegen der Feuchtigkeit spendenden Wirkung des Cholins sind die Verbindungen insbesondere zur Verwendung in Haütpflegeprodukten geeignet.

Ebenso können sie als waschaktive Komponente in Wasch- und Reinigungsmitteln oder als Emulgator zum Stabilisieren von Emulsionen verwendet werden. Weiterhin kommt der Einsatz in technischen Anwendungen, speziell im Bereich der Biochemie oder Pharmazie in Betracht, beispielsweise um SDS in der Gelelektrophorese oder in pharmakologischen Zytotoxizitätstests zu ersetzen.

Die erfindungsgemäßen bzw. erfindungsgemäß eingesetzten Cholinverbindungen unterscheiden sich von konventionellen Alkalimetall-Sulfat-Tensiden dadurch, dass ein quaternäres Ammonium-Gegenion biologischen Ursprungs, nämlich Cholin, zum Einsatz kommt. Sie können auf Basis nachwachsender Rohstoffe wie Fettsäuren produziert werden, weisen eine gute biologische Abbaubarkeit und Hautverträglichkeit auf und haben zudem im Vergleich zu Alkalimetall-Alkylsulfaten eine erhöhte Löslichkeit und verringerte Salzempfindlichkeit.

Eine bevorzugte Ausführungsform sind die erfindungsgemäßen Cholinverbindungen mit langkettigem R (14 oder mehr Kohlenstoffatome, z.B. 14 bis 24 Kohlenstoffatome). R ist dabei bevorzugterweise linear und gesättigt oder einfach ungesättigt und hat 14 bis 24 Kohlenstoffatome, insbesondere 16 bis 18 Kohlenstoffatome.

Diese Verbindungen können beispielsweise auch aus C14 bis C24-Fettsäuren (z.B. Myristinsäure, Palmitinsäure, Stearinsäure, Ölsäure oder Behensäure) oder den entsprechenden Fettalkoholen, welche in natürlichen Rohstoffen wie Palmöl, Kokospalmöl, Getreidekeimöl oder Baumwollsaatöl vorkommen, hergestellt werden. Im Gegensatz zu ihren Alkalimetall-Analoga weisen sie bei Raumtemperatur eine hohe Löslichkeit auf und können daher vorteilhaft als Tensid eingesetzt werden. Bei den Alkalimetall-Analoga mit langkettigem R ist dies wegen der geringen Löslichkeit nur sehr eingeschränkt der Fall.

Ein Vorteil der erfindungsgemäßen langkettigen Tenside liegt in ihrer erhöhten Oberflächenaktivität. So lässt sich mit einem kürzerkettigen Tensid wie SDS oder dem erfindungsgemäßen ChDS die Oberflächenspannung bei Raumtemperatur auf etwa 36-37 mN/m herabsetzen, wie im Ausführungsbeispiel gezeigt. Mit den langkettigen Verbindungen sind jedoch deutlich niedrigere Werte möglich, beispielsweise bis zu 20 mN/m. Folglich stellt die vorliegende Erfindung auch Verbindungen bereit, mit denen sich bei Raumtemperatur eine besonders niedrige Oberflächenspannung von beispielsweise bis zu 20 mN/m erreichen lässt.

Die oben erläuterten Vorteile in Bezug auf Erhöhung der Löslichkeit sowie Verbesserung der Salzempfindlichkeit und Hautverträglichkeit gegenüber den Alkalimetall-Analoga gelten nicht nur für die einfachen erfindungsgemäßen Cholin-Sulfat-Verbindungen, in denen A für Sauerstoff steht, sondern insbesondere auch für die alkoxylierten Verbindungen mit einer Gruppe der Formel - -[O-B]ₙ-O- als A. Aufgrund des zusätzlichen löslichkeitserhöhenden Effekts der Oxyalkylengruppen lassen sich bei den alkoxylierten Verbindungen besonders hohe Löslichkeiten erzielen.

Als Vorteile der erfindungsgemäßen Verbindungen gegenüber Cholin-Carboxylatseifen lassen sich ebenfalls die erhöhte Löslichkeit und verringerte Salzempfindlichkeit anführen. Ein weiterer Vorteil liegt in der verbesserten Handhabbarkeit: Im Gegensatz zu den Cholin-Carboxylatseifen ist ChDS ein nichthygroskopisches weißes Pulver, mit dem einfach an Luft gearbeitet werden kann.

Im Ausführungsbeispiel wird exemplarisch gezeigt, dass Cholindodecylsulfat als erfindungsgemäß verwendetes Cholinalkylsulfat weniger toxisch und reizend ist als das entsprechende Alkalimetall-Analogon SDS.

Die deutlich erhöhte Löslichkeit der erfindungsgemäßen bzw. erfindungsgemäß verwendeten Cholinverbindungen im Vergleich zu den entsprechenden Alkalimetallsulfaten ist ebenfalls im Ausführungsbeispiel demonstriert. Es wurde gefunden, dass beispielsweise Cholindodecylsulf at (ChDS) in Wasser löslich ist bis unter 0°C. Im Vergleich dazu löst sich Natriumdodecylsulfat (SDS) typischerweise erst bei 12-25°C (J. K. Weil, F. D. Smith, A. J. Stirton, R. G. Bistline Jr., J. Am. Oil Chem. Soc., 1963, 40 (10), 538-541). Wie oben beschrieben, ermöglicht diese verbesserte Wasserlöslichkeit der erfindungsgemäßen bzw. erfindungsgemäß verwendeten Cholinverbindungen im Vergleich zu den entsprechenden Natriumsalzen auch den Gebrauch von längerkettigen Homologen wie z.B. Myristylsulfat oder Stearylsulfat, die erhöhte Oberflächenaktivität aufweisen.

Im Gegensatz zu den entsprechenden Natriumsalzen sind die erfindungsgemäßen bzw. erfindungsgemäß verwendeten Cholinverbindungen wie beispielsweise ChDS auch in organischen Lösungsmitteln wie Chloroform, Aceton, Acetonitril oder Dimethylsulfoxid gut löslich, in denen SDS nur geringe Löslichkeit zeigt.

Außerdem bindet Cholin stärker an Sulfate als Alkalimetallionen oder NH₄⁺. Auf Grund dessen sind die erfindungsgemäßen bzw. erfindungsgemäß verwendeten Cholinverbindungen weniger salzempfindlich, wie im Beispiel gezeigt.

Die erfindungsgemäßen bzw. erfindungsgemäß verwendeten Cholinverbindungen können beispielsweise auf den folgenden drei Wegen aus den entsprechenden kommerziell erhältlichen Schwefel-Verbindungen oder deren Salzen und kommerziell erhältlichen Cholinsalzen hergestellt werden:_

### 1. Direkte Neutralisation der Alkylschwefelsäure

Die Synthese der entsprechenden Schwefelsäure-Verbindungen R-A-SO₃H ist in zahlreichen Patenten und anderen Veröffentlichungen dokumentiert, beispielsweise in DE 3503986, US 3069242 oder DE 4212086, und wird auch großtechnisch weithin durchgeführt. Die Säuren werden dann typischerweise mit Natriumhydroxid neutralisiert, um Natrium-Sulfat-Tenside (R-A-SO₃Na) zu erhalten.

Analog dazu können die Säuren mit Cholinhydroxid (ChOH) neutralisiert werden, um erfindungsgemäße Cholinsulfat-Verbindungen gemäß Gleichung (a) zu gewinnen:
a)

   R-A-SO₃H + ChOH → R-A-SO₃Ch + H₂0

### 2. Umsalzung

Alternativ dazu können die erfindungsgemäßen bzw. erfindungsgemäß verwendeten Cholinverbindungen auch gemäß Gleichung b) durch Umsetzung der entsprechenden Alkalimetall- oder Ammonium- (NH₄⁺)-Verbindungen (R-A-SO₃M) mit Cholinhalogeniden (ChX) in wässriger Lösung hergestellt werden. Dabei ist M ein Alkalimetall-Kation oder NH₄⁺ und X ist ein Halogenid-Ion:.
b)

R-A-SO₃M + ChX → R-A-SO₃Ch + MX

Die so erhaltenen Cholinverbindungen können anschließend aus der wässrigen Lösung z.B. mit Chloroform extrahiert werden.

### 3. Ionenaustausch

Schließlich können die erfindungsgemäßen bzw. erfindungsgemäß verwendeten Cholinverbindungen auch durch Ionenaustausch synthetisiert werden. Hierzu kann zum Beispiel ein starker Kationenaustauscher verwendet werden, der zunächst mit einer Cholinsalz- oder Cholinhydroxidlösung behandelt wird. Der cholinbeladene Ionenaustauscher wird anschließend mit einer Lösung des entsprechenden Alkalimetall- oder Ammoniumsulfats (R-A-SO₃M) umgesetzt.

### Ausführungsbeispiel

### Herstellung von ChDS

Cholindodecylsulfat (ChDS) wurde aus einer wässrigen SDS-Lösung (Merck, Min. 99%) durch Ionenaustausch unter Verwendung eines stark sauren Ionenaustauschers (Merck Type I) hergestellt. Hierzu wurde der Ionenaustauscher zuerst mit Millipore Wasser und dann, um ihn in die saure Form zu überführen, mit 1 N HCl gespült. Daraufhin wurde er mit 0.1 N Cholinhydroxid-Lösung (Taminco) neutralisiert und wiederum mehrmals mit Wasser gewaschen. Um einen vollständigen Ionenaustausch zu garantieren, wurde die Konzentration der SDS -Lösung so gewählt, dass die Menge der auszutauschenden Na⁺-Ionen vierfach unterhalb der maximalen Austauschkapazität lag. Anschließend konnte das reine Tensid ChDS als weißes Pulver durch Verdampfung des Wassers gewonnen werden.

### Zytotoxizitätsbestimmung

Die Zytotoxizität und Hautverträglichkeit von ChDS im Vergleich zu anderen gängigen anionischen Tensiden wurden mittels in-vitro Tests an HeLa-Zellen (humane Gebärmutterhalskrebszellen) und SK-MHL 28 Zellen (humane Melanom-Zellen) getestet. Dieses etablierte in vitro Verfahren ist in N. Vlachy, D. Touraud, J. Heilmann, W. Kunz, Colloids and Surfaces B: Biointerfaces, 2009, 70, 278 - 280 beschrieben und bietet eine gute Alternative zu den in vivo Draize-Augenirritationstests.

Die Zellen wurden von der American Type Culture Collection (ATCC) zur Verfügung gestellt. Die Kultivierung der HeLa-Zellen erfolgte in Earle's minimum essential medium (MEM) mit 0.85 g/l Natriumhydrogencarbonat, zusätzlich versetzt mit fötalem Kälberserum (10%), L-Glutamin (2mM), nichtessentiellen Aminosäuren (1%), Amphotericin B (0.4 g/ml) und Penicillin G/Streptomycin Sulfat (100u/ml). Die SK - MEL 28 - Zellen wurden in einer 1:1 Mischung aus Dulbecco's MEM mit 4.5 g/l Natriumhydrogencarbonat sowie LGlutamin und Pyrovat und HAM'S F-12 Medium mit 1.176 g/l Natriumhydrogencarbonat und stabilem Glutamin, versetzt mit 10% fötalem Kälberserum, kultiviert.

Die Inhibitionskonzentration an Testsubstanz, welche zu einer Reduktion an lebenden Zellen um 50% im Vergleich zu einer unbehandelten Kontrolle führt (IC 50), wurde via MTT-Assay nach Mosmann festgestellt.

Hierzu wurden in der ersten Spalte einer 96-Well-Platte je 15 µl an wässriger Testsubstanzlösung mit 135 µl Medium durchmischt. Je 75 µl dieser Mischung wurden abgenommen und in der zweiten Spalte mit 75 µl Medium verdünnt. Diese Prozedur wurde bis zur vollständigen Füllung der Platte von Spalte zu Spalte wiederholt. Anschließend wurden in jedem Well 11250 Zellen in 75 µl Medium angesät. Die so vorbereiteten Platten wurden 72 Stunden bei 37°C im Brutschrank aufbewahrt.

Danach wurden 15 µl MTT - Lösung zu jedem Well gegeben und für weitere 4 Stunden inkubiert. Im Anschluss wurde die überstehende Lösung abgetrennt und der reduzierte MTT-Bodensatz jeweils mit 150 µl 10%iger Natrumdodecylsulfat-Lösung versetzt. Nachdem die Platten über Nacht unter Lichtausschluss stehen gelassen wurden, wurde die Absorption der Lösungen bei 560 nm mittels einem TECAN Spectra Fluor Plus microplate reader bestimmt. Nach Mittelung an zwei Einzelmessungen konnte aus den sich ergebenden Dosis-Wirkungskurven der entsprechende IC 50 Wert errechnet werden. Angegebene Werte der Inhibitionskonzentration errechnen sich aus mindestens 5 unabhängigen Messungen.

Die folgenden Testsubstanzen wurden untersucht:
Cholindodecylsulfat (ChDS) als Beispiel für ein erfindungsgemäß verwendetes Tensid, das entsprechende Natriumsalz, Natriumdodecylsulfat (SDS), sowie Kaliumlaurat (KC12), Natriumlaurat (NaC12) und Cholinlaurat (ChC12) als Carboxylat-Tenside zum Vergleich. Die gemessenen IC50-Werte sind in der folgenden Tabelle 1 angegeben. Hohe Werte stehen für geringe Toxizität.

**Tabelle 1**

| Tensid | IC50 [mmol/l] | |
|---|---|---|
| | HeLa | SK-MEL 28 |
| ChDS | 0.404 ± 0.004 | 0.43 ± 0.02 |
| SDS | 0.27 ± 0.01 | 0.32 ± 0.03 |
| KC12 | 0.234 ± 0.005 | 0.40 ± 0.01 |
| NaCl2 | 0.23 ± 0.01 | 0.42 ± 0.01 |
| ChCl2 | 0.305 ± 0.006 | 0.36 ± 0.01 |

Wie die Ergebnisse zeigen, liegen die Toxizitäten des erfindungsgemäß verwendetes Tensids ChDS, des Cholinlaurats und der herkömmlichen anionischen Tenside, welche generell als nichtgiftig eingestuft werden, in derselben Größenordnung. Jedoch weist ChDS von den untersuchten Tensiden sowohl bei HeLa- als auch bei SK-MEL 28-Zellen den höchsten IC50-Wert auf und übertrifft sowohl SDS als auch ChCl2. Somit zeigt ChDS die geringste Toxizität.

### Löslichkeit und Salzempfindlichkeit

Als Maß für die Löslichkeit und Salzempfindlichkeit wurden die Krafft-Punkte in Abwesenheit von Salzen sowie bei unterschiedlichen Salzkonzentrationen herangezogen.

Allgemein kann die Krafft-Temperatur eines Tensids als die Löslichkeitstemperatur einer 1 Gew. %igen Lösung definiert werden, da die CMC generell weit unterhalb dieser Konzentration liegt (R. G. Laughlin, The Aqueous Phase Behavior of Surfactants, Academic Press, San Diego, 1994, p. 108). Dementsprechend wurde zur Bestimmung der Krafft-Temperatur von ChDS eine 1 gew. - %ige Lösung eingewogen. Zur Untersuchung des Einflusses von Salzen auf den Krafft-Punkt wurden 1 gew% - igen Lösungen von ChDS verschiedene Mengen an Natriumchlorid (Merck, p.a.) bzw. Cholinchlorid (Sigma-Aldrich, min. 98%) zugesetzt und die zugehörige Löslichkeitstemperatur gemessen.

Präzise Löslichkeitstemperaturen wurden aus Trübungsmessungen gemäß des in S. Schrödle, R. Buchner, W. Kunz, Fluid Phase Equilib., 2004, 216, 175-182 beschriebenen Verfahrens erhalten. Hierzu wurden alle untersuchten Proben bis zum Auftreten eines Niederschlags abgekühlt und anschließend mit einer Rate von 5°C pro Stunde wieder aufgeheizt.

Tabelle 2 zeigt die gemessenen Krafft-Temperatur von ChDS sowie entsprechende Literaturwerte der Vergleichssubstanzen:

**Tabelle 2:**

| | T_{Kr} |
|---|---|
| ChDS | 0°C |
| SDS | 16°C⁽¹⁾ |
| KDS | 40°C⁽¹⁾ |
| NaCl2 | 25°C⁽²⁾ |
| KC12 | 10°C⁽³⁾ |
| ChCl2 | <0°C⁽³⁾ |
| ChCl4 | 0°C⁽³⁾ |
| Referenzen: (1): Helmut W. Stache, Anionic Surfactants, Surfactants Science Series Vol. 56, CRC, 1995 (2) J. W. McBain, W. W. Lee, Oil Soap, 1943, 20, 17 - 25 (3) R. Klein, D. Touraud, W. Kunz, Green Chemistry, 2008, 10, 433 - 435 | |

Wie der Tabelle zu entnehmen ist, weist ChDS eine deutlich niedrigere Krafft-Temperatur und damit eine erhöhte Löslichkeit im Vergleich zu SDS und KDS (Kaliumdodecylsulfat) auf. SDS hat wiederum eine deutlich niedrigere Krafft-Temperatur als KDS, was die erwartungsgemäße Verringerung der Löslichkeit mit zunehmender Größe des Kations demonstriert.

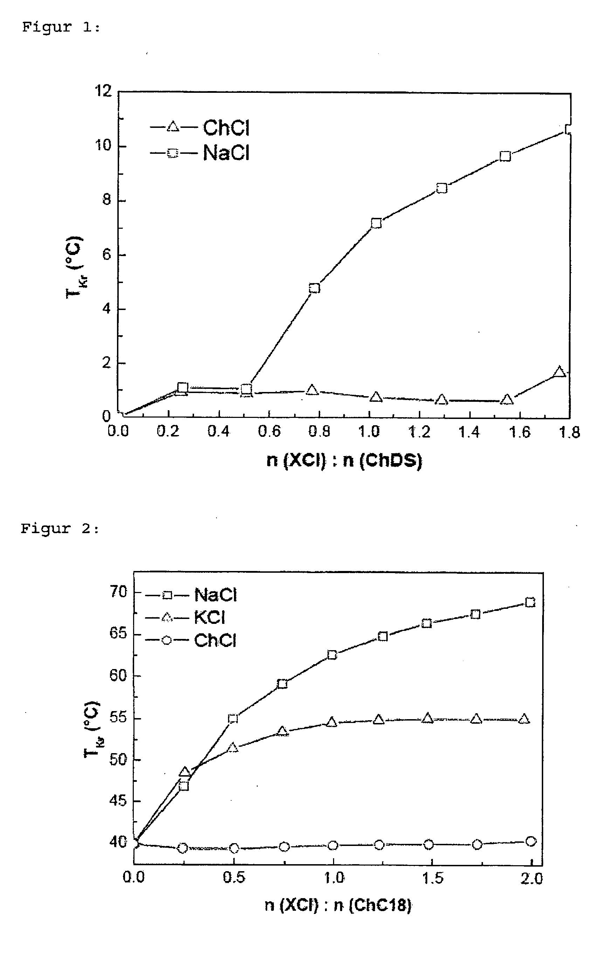

Die Salzempfindlichkeit wurde durch Messung der Krafft-Temperatur bei unterschiedlichen Salzkonzentrationen untersucht. In Figur 1 ist die Krafft-Temperatur von ChDS gegen die Konzentration an NaCl und ChCl aufgetragen. Ein NaCl:ChDS-Verhältnis von 1:1 bewirkt einen Anstieg um etwa 7°C.

Zum Vergleich zeigt Figur 2 die Krafft-Temperatur von Cholinstearat (ChCl8) in Abhängigkeit von der Salzkonzentration. Hier bewirkt ein NaCl:ChC18-Verhältnis von 1:1 einen Anstieg um etwa 23°C.

### Oberflächenaktive Wirkung

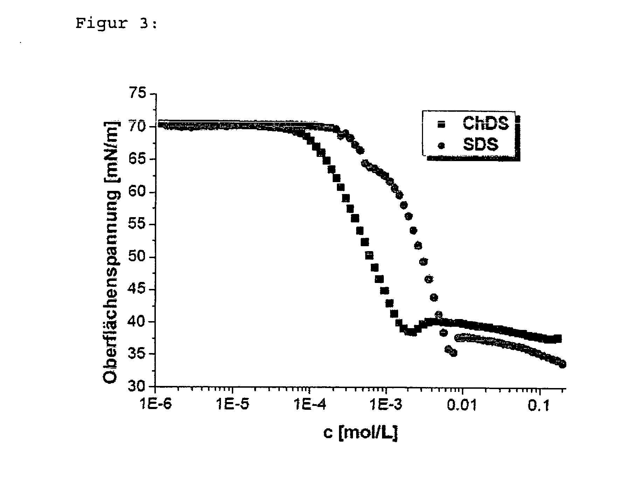

Die Herabsetzung der Grenzflächenspannung von Wasser durch Zugabe an Tensid ist ein wichtiges Maß für die Effizienz eines Tensids. Die Oberflächenspannung von wässrigen ChDS- und SDS- Lösungen wurde anhand der Ringmethode mit einem Krüss Tensiometer K100 vermessen. Figur 3 zeigt die gemessene Grenzflächenspannung bei einer Temperatur von 25°C in Abhängigkeit von der Konzentration.

Wie in Figur 3 gezeigt, reduziert das erfindungsgemäß verwendete Tensid die Grenzflächenspannung von Wasser von ca. 70 mN/m auf ca. 37 mN/m, was in der Größenordnung von SDS (ca. 36 mN/m) liegt.

## Patentansprüche

1. Cholinverbindung der Formel
R-A-SO₃⁻ [(H₃C)₃N-CH₂-CH₂-OH] ⁺
worin A für ein Sauerstoffatom oder eine Gruppe der Formel -[O-B]ₙ-O- steht, wobei B eine Alkylengruppe mit 2 bis 4 Kohlenstoffatomen darstellt und n für eine Zahl von 1-20 steht;
und R für eine gesättigte oder ungesättigte, lineare oder verzweigte Alkylgruppe mit 8 bis 30 Kohlenstoffatomen steht,
wobei β-Hydroxyethyltrimethylammoniumdodecylsulfat ausgenommen ist.

2. Cholinverbindung gemäß Anspruch 1, worin R für eine gesättigte oder ungesättigte, lineare oder verzweigte Alkylgruppe mit 8 bis 24 Kohlenstoffatomen steht.

3. Cholinverbindung gemäß Anspruch 2, worin R für eine gesättigte oder ungesättigte, lineare oder verzweigte Alkylgruppe mit 8 bis 20 Kohlenstoffatomen steht.

4. Cholinverbindung gemäß Anspruch 3, worin R für eine gesättigte oder einfach ungesättigte, lineare Alkylgruppe mit 12 bis 18 Kohlenstoffatomen steht.

5. Cholinverbindung gemäß Anspruch 4, worin R aus n-Dodecyl oder n-Tetradecyl ausgewählt ist.

6. Cholinverbindung gemäß Anspruch 2, worin R für eine gesättigte oder einfach ungesättigte, lineare Alkylgruppe mit 14 bis 24 Kohlenstoffatomen steht.

7. Cholinverbindung gemäß einem der Ansprüche 1-6, worin A ein Sauerstoffatom ist.

8. Cholinverbindung gemäß einem der Ansprüche 1-6, worin A für die Gruppe der Formel -[O-B]ₙ-O- steht und B eine Ethylengruppe -CH₂-CH₂-, eine 2-Propylengruppe -CH(CH₃) -CH₂- oder eine Mischung daraus ist.

9. Verwendung der Cholinverbindung der Formel
R-A-SO₃⁻ [(H₃C)₃N-CH₂-CH₂-OH]⁺
worin A für ein Sauerstoffatom oder eine Gruppe der Formel -[O-B]ₙ-O- steht, wobei B eine Alkylengruppe mit 2 bis 4 Kohlenstoffatomen darstellt und n für eine Zahl von 1-20 steht,
und R für eine gesättigte oder ungesättigte, lineare oder verzweigte Alkylgruppe mit 8 bis 30 Kohlenstoffatomen steht,
als Tensid.

10. Verwendung gemäß Anspruch 9, wobei R, A und B wie in einem der Ansprüche 2 bis 8 definiert sind.

11. Verwendung gemäß Anspruch 9 oder 10 in Haarpflegeprodukten, Körperpflegeprodukten oder Hautpflegeprodukten.

12. Verwendung gemäß Anspruch 9 oder 10 in Wasch- und Reinigungsmitteln.

13. Verwendung gemäß Anspruch 9 oder 10 zur Stabilisierung von Emulsionen.

## Claims

1. Choline compound of the formula
R-A-SO₃⁻ [(H₃C)₃N-CH₂-CH₂-OH] ⁺
wherein A represents an oxygen atom or a group of the formula - [O-B]ₙ-O-, wherein B is an alkylene group having 2 to 4 carbon atoms and n represents a number from 1-20;
and R represents a saturated or unsaturated, linear or branched alkyl group having 8 to 30 carbon atoms,
wherein β-hydroxyethyltrimethylammonium dodecyl sulphate is excluded.

2. Choline compound according to claim 1, wherein R represents a saturated or unsaturated, linear or branched alkyl group having 8 to 24 carbon atoms.

3. Choline compound according to claim 2, wherein R represents a saturated or unsaturated, linear or branched alkyl group having 8 to 20 carbon atoms.

4. Choline compound according to claim 3, wherein R represents a saturated or simply unsaturated, linear alkyl group having 12 to 18 carbon atoms.

5. Choline compound according to claim 4, wherein R is selected from n-dodecyl or n-tetradecyl.

6. Choline compound according to claim 2, wherein R represents a saturated or simply unsaturated, linear alkyl group having 14 to 24 carbon atoms.

7. Choline compound according to one of claims 1-6, wherein A is an oxygen atom.

8. Choline compound according to one of claims 1-6, wherein A represents the group of the formula - [O-B]ₙ-O- and B is an ethylene group -CH₂-CH₂-, a 2-propylene group -CH(CH₃)-CH₂- or a mixture thereof.

9. Use of the choline compound of the formula
R-A-SO₃⁻ [(H₃C)₃N-CH₂-CH₂-OH]⁺
wherein A represents an oxygen atom or a group of the formula - [O-B]ₙ-O-, wherein B is an alkylene group having 2 to 4 carbon atoms and n represents a number from 1-20,
and R represents a saturated or unsaturated, linear or branched alkyl group having 8 to 30 carbon atoms,
as a surfactant.

10. Use according to claim 9, wherein R, A and B are defined as in one of claims 2 to 8.

11. Use according to claim 9 or 10 in hair care products, personal hygiene products or skin care products.

12. Use according to claim 9 or 10 in detergents and cleaning agents.

13. Use according to claim 9 or 10 for the stabilisation of emulsions.

## Revendications

1. Composé de choline de formule
R-A-SO₃⁻ [(H₃C)₃N-CH₂-CH₂-OH]⁺
dans laquelle A représente un atome d'oxygène ou un groupe de formule -[O-B]ₙ-O-, dans laquelle B représente un groupe alkylène ayant de 2 à 4 atomes de carbone et n représente un nombre de 1 à 20 ;
et R représente un groupe alkyle saturé ou insaturé, linéaire ou ramifié ayant de 8 à 30 atomes de carbone,
le dodécylsulfate de β-hydroxyéthyltriméthylammonium en étant exclu.

2. Composé de choline selon la revendication 1, dans lequel R représente un groupe alkyle saturé ou insaturé, linéaire ou ramifié ayant de 8 à 24 atomes de carbone.

3. Composé de choline selon la revendication 2, dans lequel R représente un groupe alkyle saturé ou insaturé, linéaire ou ramifié ayant de 8 à 20 atomes de carbone.

4. Composé de choline selon la revendication 3, dans lequel R représente un groupe alkyle linéaire saturé ou mono-insaturé ayant de 12 à 18 atomes de carbone.

5. Composé de choline selon la revendication 4, dans lequel R est choisi parmi le n-dodécyle ou n-tétradécyle.

6. Composé de choline selon la revendication 2, dans lequel R représente un groupe alkyle linéaire saturé ou mono-insaturé ayant de 14 à 24 atomes de carbone.

7. Composé de choline selon l'une des revendications 1 à 6, dans lequel A est un atome d'oxygène.

8. Composé de choline selon l'une des revendications 1 à 6, dans lequel A représente le groupe de formule -[O-B]ₙ-O- et B est un groupe éthylène -CH₂-CH₂-, un groupe 2-propylène -CH(CH₃)-CH₂- ou un mélange de ceux-ci.

9. Utilisation du composé de choline de formule
R-A-SO₃⁻ [(H₃C)₃N-CH₂-CH₂-OH]⁺
dans laquelle A représente un atome d'oxygène ou un groupe de formule -[O-B]ₙ-O-, dans laquelle B représente un groupe alkylène ayant de 2 à 4 atomes de carbone et n représente un nombre de 1 à 20,
et R représente un groupe alkyle saturé ou insaturé, linéaire ou ramifié ayant de 8 à 30 atomes de carbone,
comme tensioactif.

10. Utilisation selon la revendication 9, dans laquelle R, A et B sont définis comme dans l'une des revendications 2 à 8.

11. Utilisation selon la revendication 9 ou 10 dans des produits de soin capillaire, des produits de soin corporel ou des produits de soin cutané.

12. Utilisation selon la revendication 9 ou 10 dans des lessives ou des détergents.

13. Utilisation selon la revendication 9 ou 10 pour la stabilisation des émulsions.
